# EUROPEAN PATENT SPECIFICATION

(11) **EP 4 223 288 B1**
(45) Date of publication and mention of the grant of the patent: **06.03.2024**
(21) Application number: 22020035.6
(22) Date of filing: 07.02.2022
(51) Int. Cl.: A61K 31/165, A61K 31/198, A61K 31/4439, A61K 45/06, A61P 25/16, A61K 9/00, A61K 9/16, A61K 9/19, A61K 9/20, A61K 47/18, A61K 9/50

(54) **NOVEL KIT OF PHARMACEUTICAL PREPARATIONS FOR THE TREATMENT OF PARKINSON'S DISEASE**
NEUER KIT VON PHARMAZEUTISCHEN PRÄPARATEN ZUR BEHANDLUNG VON MORBUS PARKINSON
NOUVEAU KIT DE PRÉPARATIONS PHARMACEUTIQUES POUR LE TRAITEMENT DE LA MALADIE DE PARKINSON

(43) Date of publication of application: 09.08.2023
(73) Proprietor: Berlirem GmbH, 14195 Berlin (DE)
(72) Inventor: HÜMPEL, Michael, 23564 Lübeck (DE); TACK, Johannes, 13595 Berlin (DE); PALLA, Dirk, 13187 Berlin (DE); PALLA, Heinz, 10557 Berlin (DE); BRÄUTIGAM, Matthias, 10629 Berlin (DE)
(74) Representative: Jungblut & Seuss

(56) References cited:
- WO-A1-2016/155888
- WO-A2-2012/079072
- WO-A2-2013/184646
- US-A- 5 607 969
- KOLLER W. C. ET AL: "Immediate-release and controlled-release carbidopa/levodopa in PD : A 5-year randomized multicenter study", NEUROLOGY, vol. 53, no. 5, 1 September 1999 (1999-09-01), pages 1012-1012, XP055935253, US ISSN: 0028-3878, DOI: 10.1212/WNL.53.5.1012 Retrieved from the Internet: URL:https://n.neurology.org/content/neurol ogy/53/5/1012.full.pdf>
- WYRWA RALF: "A new type of highly soluble L-DOPA ester for continuous subcutaneous application in Parkinson's disease", MOVEMENT DISORDERS CLINICAL PRACTICE, vol. 7, no. S2, 1 March 2020 (2020-03-01), page S21, XP055934354, ISSN: 2330-1619, DOI: 10.1002/mdc3.12923 Retrieved from the Internet: URL:https://onlinelibrary.wiley.com/doi/fu ll-xml/10.1002/mdc3.12923>

## Description

### Summary

The invention is set out in the appended set of claims. Any subject-matter falling outside the scope of the claims is provided for information purposes only.

The present invention relates to a kit for the treatment of Parkinson patients with motor complications in need of Continuous Dopaminergic Stimulation. Levodopa (LD) is applied via continuous subcutaneous infusion combined with an almost complete inhibition of the peripheral levodopa metabolism to achieve Continuous Dopaminergic Stimulation.

The effective inhibition of peripheral levodopa metabolism allows a marked reduction of the necessary levodopa (or equivalent of prodrug) doses to 300 - 600 mg/day, which is, however, sufficient to reach high levodopa plasma levels of 1500 to 3000 ng/ml.

### BACKGROUND - Field/Prior Art

**Field:** Parkinson's disease (PD) is characterized by the inability of the dopaminergic neurons in the substantia nigra of the brain to produce and store the neurotransmitter dopamine. Parkinson's disease impairs motor skills, cognitive processes and autonomic functions.

In the healthy brain, dopaminergic neurons in the basal ganglia normally fire in a continuous manner, maintaining the striatal dopamine concentration at a relatively stable level. Striatal dopamine concentrations are relatively constant, and there is continuous activation of dopamine receptors. There is evidence that in Parkinson's disease, dopaminergic treatment with short-acting oral drugs produces a discontinuous stimulation, inducing an intermittent pulsatile activation of the striatal receptors. According to present understanding, it is likely that the drug-induced oscillations of the dopamine level in the striatum contribute to the development of complications, such as motor fluctuations and dyskinesias.

Continuous Dopaminergic Stimulation is, therefore, the therapeutic aim for the management of Parkinson's disease proposing that Continuous Dopaminergic Stimulation - as opposed to discontinuous or pulsatile, stimulation of striatal dopamine receptors - will treat, delay or prevent levodopa-related motor complications.

**Prior art:** Oral levodopa treatment is still the gold standard of any pharmacologic anti-Parkinson therapy and has historically always been administered in a fixed combination with an AADC-inhibitor. This treatment paradigm does not allow an optimization of the AADC-inhibitor dose to attain stronger inhibition of peripheral DOPA decarboxylation. In the case of carbidopa (CD), the combination contains 4 or 10 parts of levodopa and 1 part of carbidopa. In the case of benserazide (BZ), only formulations with a 4 : 1 ratio are available, because the potency of benserazide is half of carbidopa (Nishikawa et al., 2020, Iwaki et al, 2014, Greenacre, 1976). The half-life of both AADC-inhibitors is short and around 1 hour. The maximum standard daily oral treatment dose is around 1,000 mg of levodopa and 250 mg carbidopa or benserazide (c.f. package insert Sinemet (levodopa/carbidopa) or Madopar (levodopa/benserazide)). The package insert of Sinemet cautions against higher daily dose than 200 mg carbidopa because of the possible risk, that penetration of carbidopa into the CNS may cause an unwanted inhibition of central AADC and thus a reduction of dopamine brain levels. In 2012, Brod et al. tested 3 × 150 mg carbidopa/day for four weeks, but did not detect an impairment of the therapeutic effects of levodopa, extending the safe range of Carbidopa to 3 × 150 mg/day. For benserazide Dingemanse, 1997 showed that 3 × 100-200 mg benserazide daily do not completely inhibit peripheral levodopa decarboxylation. No unexpected side effects were reported.

In recent years, COMT-inhibitors were developed to block the levodopa metabolism further. Opicapone50 mg given once a day, reaches an almost complete inhibition of COMT after 10-14 days of use and is sometimes added to the levodopa/AADC-inhibitor treatment regime. There are other minor pathways of levodopa metabolism being responsible for 10-20 % of overall peripheral levodopa metabolism (Männisto et al. 1992). Percentages refer to the total levodopa clearance (metabolism plus renal clearance) without inhibitors.

Many combination products (levodopa plus carbidopa or benserazide) are available as immediate release (IR) formulations. Due to rapid absorption and short disposition half-lives of both compounds the amplitude in plasma levels generally is high for 3-5 times administrations per day. The peak-trough levels differ by 30-40% of the mean and thereby are the main reason for unwanted changes in "on/off" times and other negative effects. Moreover, the systemic availability of levodopa shows a broad scatter because of two different processes, i.e., the variabilities in enteral absorption rate and degree, and the non-harmonized absorption process of the two components leading to additional variabilities in levodopa pharmacokinetics. In order to overcome the limitations of multiple administrations of immediate release formulations per day and to reduce the peak-trough amplitude, quite a number of alternative formulations were developed in the last decades.

Intravenous infusions: Numerous studies demonstrate that iv infusion of levodopa stabilizes its concentration in plasma and dramatically reduces or prevents development of motor complications (see, for example, Shoulson et al., Neurology 25:1144 (1975); Quinn et al., Lancet. 2:412 (1982) Nutt et al., N Engl J. Med. 310:483 (1984)). Although these studies are the proof of concept that Continuous Dopaminergic Stimulation must be the final goal of a successful antiparkinsonian therapy, the i.v. approach is not feasible however due to the low solubility of levodopa (high daily iv infusion volumes of 100 ml/h and more are necessary), besides the difficulties and risks of a continuous intravenous infusion in an ambulant environment.

Oral modified release formulations: Various oral modified release formulations were developed and introduced to the clinic to compensate for the short elimination half-life of levodopa and to reduce its pulsatile trough and peak levels. However, despite tremendous efforts, the goal of Continuous Dopaminergic Stimulation has never been achieved by any kind of oral modified release formulations. The problem of fluctuating levodopa plasma levels (trough/peak ratio of about 30-40% of mean) is further aggravated by delayed gastric emptying which is common in Parkinson's disease patients and contributes to fluctuations in levodopa uptake from the bowel. As a result, this leads to largely unpredictable motor responses observed with orally applied levodopa (Statement from Nyholm et al, 2013).

Due to the urgent need of applying any effective treatment for late-stage Parkinson patients, the intrajejunal infusion of levodopa/carbidopa gel (fixed levodopa/carbidopa ratio of 4 : 1) was developed. With this procedure a gel formulation is delivered directly (for 16h/day) into the proximal jejunum via a percutaneous endoscopic gastrojejunostomy (PEG-J) tube (c.f. package leaflet Duodopa). Very high plasma levels of levodopa can be achieved, as it is needed for the treatment of late-stage Parkinson's disease cases. However, respective levels show a considerable scatter according to absorption variabilities. The treatment is a highly invasive, unpleasant and cumbersome therapeutic method and bears the risk of adverse effects mainly from the surgical procedure. Moreover, it is difficult for the patient to manage, so that most - even severely disabled - Parkinson patients shy away from this ultima ratio treatment. In addition, it is becoming increasingly evident, that the risk of peripheral neuropathies rises with higher levodopa doses (Romagnolo et al. 2018).

Subcutaneous infusions of levodopa/carbidopa preparations: Therefore, in the last 10 years other strategies have evolved and are currently in clinical evaluation for their usefulness in the treatment of M. Parkinson. In particular, subcutaneous infusions of levodopa/carbidopa or levodopa/carbidopa esters are being tested concerning their suitability to reach sufficiently high and constant levodopa plasma levels to justify their clinical use. It has to be noted in this context that, in order to effectively treat severe Parkinson's disease stages with motor complications, steady-state levodopa plasma levels of at least 1500 ng/ml have to be established, whereas more advanced late-stage Parkinson's disease patients with postural instability even require levodopa plasma levels of 2000 - 3000 ng/ml and higher.

These minimum requirements for sufficiently high and constant levodopa plasma concentrations (as known from present pharmacologic therapy of varying severity grades of Parkinson's disease) have to be seen in relation to the low levodopa plasma levels reported from clinical studies of two subcutaneous levodopa therapies currently in development:

### NeuroDerm Pharma's ND-0612:

Caracao (2012) presented ND0612L (L = low) as a novel liquid formulation of levodopa 60mg/ml made soluble in arginine (US 4,409,233) plus carbidopa 14 mg/ml (recently reduced to 7 mg/ml), the infusion volume/day was around 5 ml, i.e., 300 mg/day levodopa were applied. With this dose, levodopa plasma levels of around 400-500 ng/ml were reached. The development of this formulation is meanwhile abandoned.

According to the NeuroDerm patent (WO2012/066538)¹, both levodopa and carbidopa were made soluble by 1:1.8 molar arginine with a final pH of 9.1 to 9.8, no further adjuvants are mentioned in the patent. Table 1 shows that the molar load of the ND-0612 solution is around 1 mol/l which is above the maximally acceptable skin tolerability² for a 24h infusion and the high pH (around 9.5) of the formulation may further limit skin tolerability.
¹ 1. A pharmaceutically acceptable composition having a pH of about 9.1 to about 9.8 at 25°C, comprising: active components comprising carbidopa and at least about 4% by weight levodopa; arginine and optionally meglumine.
2. The pharmaceutically acceptable composition of claim 1, wherein the molar ratio of active components to the arginine is about 1:1.8 to about :3.5, or about 1:2.3.
² Some consider 500 mosmol/l as problematic: Stranz and Kastango, 2002

Neuroderm changed their development project and introduced a twin pump system for the subcutaneous infusion of the higher-dosed levodopa formulation ND0612H (High). The system consists of two separate skin infusion sites and injects 2 times 360 mg/day of levodopa. But even with this twin injection system (720 mg levodopa/day) plasma levels not higher than 800 -1000 ng/ml were reached. Borderline skin tolerability was reported - despite a daily change of infusion sites.

### AbbVie's ABBV-951:

Rosebraugh (2019) reported about ABBV-951 as a subcutaneous infusion for Parkinson patients. ABBV-951 is a mixture of levodopa phosphate ester of 240 mg/ml and carbidopa phosphate ester, the latter was recently reduced to 12 mg/ml resulting in a drug/inhibitor mass ratio of 20:1. Additional excipients were not disclosed. With a maximum infusion volume of 4 ml/day (or 960 mg Levodopa-Phosphate equivalent to 680 mg levodopa), Rosebraugh reported levodopa plasma levels of around 800 ng/ml in volunteers. Higher daily infusion doses have never been reported and Table 1 shows, that ABBV-951 also reaches the critical molar load level for skin infusions of 1 mol/l.

Summary of prior art for subcutaneous infusions: Clinically, the infusion of around 700 mg/day levodopa (or levodopa equivalents) of ND0612H or by ABBV-951 achieves levodopa plasma levels of at most 800 - 1000 ng/ml being insufficient to effectively treat moderate let alone severe Parkinsonism. Higher plasma concentrations (around 1500 ng/ml) are clearly necessary for Continuous Dopaminergic Stimulation in later stages of the disease (LeWitt, 2014). To reach the 800-1000 ng/ml levodopa plasma levels Neuroderm had to use two infusion sites with twin pump system, most probably because of limited skin tolerability of the formulation. The product is developed with an add-on oral levodopa/carbidopa dosing regimen.³
³ NCT04006210: A Clinical Trial Investigating the Efficacy, Safety and Tolerability of Continuous Subcutaneous ND0612 Infusion in Comparison to Oral IR-LD/CD in Subjects with Parkinson's Disease Experiencing Motor Fluctuations (BouNDless).

Obviously, the present strategy to combine levodopa and an AADC-Inhibitor into one subcutaneous formulation does not reach the goal of sufficiently high levodopa plasma levels. Skin tolerability problems, probably from carbidopa, limit this approach. Recently, during the development, the AADC-inhibitor carbidopa was lowered below the well-established 4:1 mass ratio (the levodopa : carbidopa ratio in ND0612 is now 8 : 1, in ABBV-951 now 20 : 1 (c.f. Table 1, attached)), but this reductions are at the expense of AADC inhibition efficacy and lead to a loss of levodopa. Even increasing the levodopa infusion-doses to around 700 mg/day could not counterbalance the incomplete AADC-inhibition. In summary, 800 - 1000 ng/ml levodopa plasma concentrations are reached with both formulations. Thus, prior art teaches that at least 700 mg/day levodopa or levodopa equivalents have to be infused subcutaneously to reach 800 -

1000 ng/ml plasma levels of levodopa which is, however, not sufficient to satisfactorily treat moderate let alone severe stages of Parkinson's disease.

These disadvantages are perceived as problematic because, due to the described limitations of the technical solutions available to date, a large number of M. Parkinson patients cannot be treated sufficiently. This applies especially to patients in later disease stages who, after initial 3 to 5 years of conventional oral levodopa therapy, develop dyskinesias and motor fluctuations which urgently require Continuous Dopaminergic Stimulation-based therapy with levodopa.

Patent application WO 2013/184646 A2 discloses treatment of Parkinson's disease by administering a combination of levodopa ester and carbidopa or benserazide wherein levodopa is subcutaneously administered and carbidopa or benserazide are orally administered.

### DESCRIPTION OF INVENTION

It has been found that according to the present invention a kit of pharmaceutical preparations, containing
a) an AADC-inhibitor selected from benserazide or carbidopa,
   wherein the AADC-inhibitor is applied in a daily dose of 300-1200mg
   wherein said AADC-inhibitor is provided as an oral Modified Release formulation,
   wherein the oral Modified Release formulation of the AADC-inhibitor is constantly releasing the AADC-inhibitor in the gastrointestinal tract in an amount of 12.5-50 mg/h over at least 6 hours, and
   wherein the oral Modified Release formulation of the AADC-inhibitor is applied 2-4 times a day
b) a liquid formulation of levodopa or levodopa ester,
   wherein the liquid formulation contains levodopa or levodopa ester in a molarity of 100-500 mmol/l
   wherein the liquid formulation is subcutaneously infused
   wherein the daily dose of levodopa or levodopa equivalent is 100-900mg
c) optionally, a pharmaceutical preparation of a COMT-inhibitor in which the COMT-inhibitor is 25-75 mg Opicapone,
can successfully be used to treat Parkinson's Disease and Restless Legs Syndrome (RLS). Further embodiments of the invention are defined in the appended claims.

The references to the methods of treatment by therapy in this description are to be interpreted as references to compounds, pharmaceutical compositions, medicaments and kits of the present invention for use in those methods.

Disclosed is furthermore a combination of (1.) a method to almost completely inhibit the peripheral levodopa metabolism by using an oral Modified Release formulation of an AADC-inhibitor (benserazide or carbidopa) plus a continuous COMT-inhibition with (2.) a method of a subcutaneous infusion of low levodopa or levodopa ester doses. The combination of both methods provides the generation of constant levodopa plasma levels up to 3000 ng/ml of levodopa. The height of steady-state plasma level is regulated by the dose/h and the individual metabolic clearance rate. Dose adjustment is triggered by clinical symptoms. Steady state is reached within 10-12 hours which can be considerably accelerated by an initial bolus infusion of individually adjustable dose.

Disclosed is therefore also a method of treating neurodegenerative diseases, in particular Parkinson's Disease or Restless Legs Syndrome (RLS) (only Parkinson's Disease and RLS are neurodegenerative diseases according to the invention) by administration to a patient
a) an AADC-inhibitor selected from benserazide or carbidopa,
   wherein the AADC-inhibitor is applied in a daily dose of 300-1200mg
   wherein said AADC-inhibitor is provided as an oral Modified Release formulation,
   wherein the oral Modified Release formulation of the AADC-inhibitor is constantly releasing the AADC-inhibitor in the gastrointestinal tract in an amount of 12.5-50 mg/h over at least 6 hours, and
   wherein the oral Modified Release formulation of the AADC-inhibitor is applied 2-4 times a day
b) a liquid formulation of levodopa or levodopa ester,
   wherein the liquid formulation contains levodopa or levodopa ester in a molarity of 100-500 mmol/l
   wherein the liquid formulation is subcutaneously infused
   wherein the daily dose of levodopa or levodopa equivalent is 100-900mg
c) optionally, a pharmaceutical preparation of a COMT-inhibitor in which the COMT-inhibitor is 25-75 mg Opicapone.

The invention allows for convenient Continuous Dopaminergic Stimulation-therapy for Parkinson patients in all severity stages of the disease, and in particular in the more advanced stages.

The term "Modified Release" used in this document means/relates to dosage forms where the rate and/or site of release of the active ingredient(s) are different from that of the immediate release dosage form administered by the same route. A modified release formulation may for example be a tablet or capsule from which the active ingredient is released slowly over several hours or it may be a liquid formulation which is applied slowly over several hour, e.g. applied by a minipump or patchpump.

### 1. Effective inhibition of peripheral levodopa-metabolism

The extensive inhibition of the peripheral levodopa metabolism is basically achieved by:
(a) a daily dose of an AADC-inhibitor (preferentially benserazide or carbidopa in doses of around 37.5 mg/h (range: 25-50 mg/h) and
(b) a Modified Release formulation administered in 2 - 4 by time equidistant doses over the total treatment time of the day.

The release of the inhibitor of AADC from the Modified Release formulation leads to constant inhibitor plasma levels and thus to an extensive blockage of decarboxylation activity. In order to counteract any lower degrees of absorption in the more distal parts of the intestinal tract, the Modified Release formulation is designed to release the inhibitor drug for up to 6-8 hours, which means that the Modified Release formulation usually needs to be taken 2-3 times a day. Only in exceptional cases the Modified Release formulation needs to be taken more than 3 times a day (e.g. 4 times). Alternatively, floating tablet devices which remain in the stomach for a predetermined delivery time or a dental pump device releasing the inhibitor of choice continuously represent examples of a variety of possible modified release formulations.

The inhibition of peripheral metabolism of levodopa is further strengthened by the application of a COMT-inhibitor, preferably by opicapone, since a once-a-day dose of e.g. 50mg/day provides optimal and continuously ongoing COMT-inhibition over 24 h.

In one embodiment (see Example 2) the subcutaneous infusion of levodopa as an aqueous solution was used to investigate the effects of oral benserazide on metabolism and pharmacokinetics of levodopa. COMT-inhibition was also part of co-treatment.

Hourly oral administrations of 12.5, 25, or 50 mg benserazide have a marked dose dependent effect on levodopa plasma levels. Surprisingly, benserazide lowered the levodopa clearance from about 10ml/min/kg (Example 1) to 2.4 ml/min/kg, thereby increasing the AUC/mg of levodopa to 80 ng*h/ml. This increase of AUC/mg levodopa is about 3-fold larger than seen from products with the established 4 :1 ratio of levodopa and inhibitor. Increases were seen with either 25 mg/h and probably optimized COMT-inhibition or with 50 mg/h with suboptimal COMT-inhibition. Benserazide therefore is the preferred embodiment of an AADC-inhibitor.

In another embodiment (see Example 3), the subcutaneous infusion of levodopa as an aqueous solution was used to investigate the effects of oral carbidopa on metabolism and pharmacokinetics of levodopa. COMT-inhibition was also part of co-treatment.

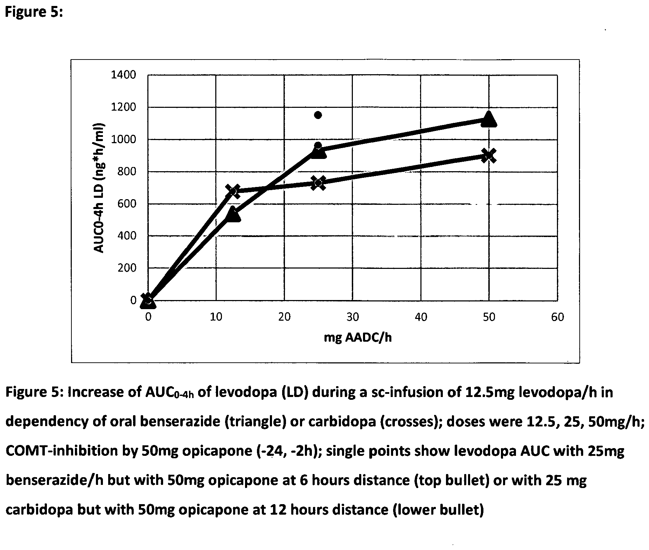

Hourly oral administrations of 12.5, 25, or 50 mg carbidopa have a dose dependent effect on levodopa plasma levels. Surprisingly, carbidopa lowered the levodopa clearance from about 10ml/min/kg (Example 1) to 3.7 ml/min/kg at a carbidopa dose of 25mg/h and to 3.1 ml/min/kg at a dose of 50mg carbidopa/h (Example 3). Even more surprising was the observation that benserazide turned out to be the more efficient AADC-inhibitor than carbidopa (identical subjects, identical test situation). In addition, as was found for benserazide, the optimized co-medication increased the levodopa plasma levels in comparison to the non-optimized opicapone treatment schedule. The observation is based on the AUC0-4h levodopa data in Table 7 (Example 3). Comparison of the respective data of carbidopa with benserazide surprisingly shows that benzerazide is at least as potent as carbidopa but shows a higher maximal inhibitory activity (figure 5), suggesting inhibition of additional minor not-well defined pathways of peripheral levodopa metabolism.

By this invention, the dose ratio known from daily oral PD treatment regime (4 dose parts levodopa to 1 dose part AADC-inhibitor) can be changed to 1 dose part sc levodopa to 1-2 dose parts oral benserazide. Generally speaking, the dose ratio between levodopa to AADC-inhibitor should be between approximately 1 : 2 and approximately 1 : 1.

Results of respective simulations show, that a Modified Release formulation linearly releasing not less than 25mg/h for 6-8 hours (to be taken 2 to 4 times a day) will be effective in the largely inhibition of AADC enzyme activity (Example 5).

This invention shows, that an effective inhibition of peripheral levodopa metabolism needs a dose of about 25 mg/h carbidopa or benserazide applied evenly over the chosen treatment time of the day. This dose is well above the previously recommended dose for carbidopa or benserazide. The AADC-inhibition needs to be accompanied by an optimized COMT-inhibition - at least for the treatment of severe PD cases.

### 2.1. Subcutaneous continuous infusion of low doses of levodopa esters (Pentadopa)

Levodopa esters may alternatively be used in the kit according to the invention. Levodopa esters and their properties and synthesis are described in EP3277660 B1. A preferred embodiment of a Levodopa ester is Pentadopa which is the levodopa pentaerythritol monoester described in EP3277660 B1. As further outlined in Example 1 of this application, Pentadopa is a highly water-soluble derivative of levodopa suitable for subcutaneous infusion. The ester is incompletely cleaved (Example 1) and without any inhibition of levodopa metabolism, about 35% of levodopa is released from the pro-drug. The inhibition of COMT increases the bioavailability of levodopa from its pro-drug to 50%. Because COMT-inhibition is a standard co-medication in Parkinson's disease, the "clinical" bioavailability of levodopa from Pentadopa is thus around 50% of dose. Other examples of Levodopa esters which may be used according to the present invention are the esters described in WO 2016/065019 A1 (assigned to Abbvie Inc.), in particular the mono- and diphosphate esters of levodopa

When the metabolism of the released levodopa is inhibited by continuous inhibition of AADC using oral 25mg benserazide/hour (plus opicapone), the sc infusion of less than 500 mg of levodopa-equivalents as Pentadopa will generate stable and reliable levodopa plasma levels of ≥ 1000 ng/ml (Example 1).

### 2.2 Subcutaneous continuous infusion of low levodopa doses (Example 4)

Studies 9 and 10 demonstrate that during long-term sc-infusion of 12.5 to 18.75mg levodopa /h under optimized AADC and COMT-inhibition, constant and stable levodopa levels of around 1500ng/ml (range 1200 to 1800 ng/ml) can be achieved. Respective levels are known to sufficiently control moderate to severe PD symptoms.

The treatment consists of an AADC-inhibitor (carbidopa or benserazide) at a dose of 25mg/h best as a Modified Release preparation and optimized COMT-inhibition preferably with opicapone (or an equivalent compound) and a sc levodopa-infusion of 300 mg/day for 16 h = 0.27mg/h/kg.

The effective inhibition of levodopa metabolism had a surprisingly high impact on the daily levodopa dose necessary to reach sufficiently high levodopa plasma levels. With the infusion of 300 mg levodopa in 16h we reached around 1500 ng/ml levodopa plasma levels, whereas prior art (ABBV-951 and ND-0612H needed 680-720 mg levodopa (or levodopa equivalents) per day to reach 800 -1000 ng/ml levodopa levels.

Keeping carbidopa or benserazide away from the subcutaneous formulation and administering the inhibitor by the oral route, as proposed by our invention, avoids irritation of subcutaneous tissue by the skin-irritating inhibitors. On the other hand, the doses of carbidopa in the sc formulations of NeuroDerm's ND0612H (3.75 mg/h) and Abbvie's ABBV-951 (1.4 mg/h), respectively, are by far too low to achieve an effective inhibition of AADC, the most important pathway in levodopa-metabolism.

### 3. Formulation

The complete inhibition of the peripheral levodopa decarboxylation and the additional COMT-inhibition allow significant dose reduction of daily levodopa doses needed to reach the desired levodopa plasma levels. The sc formulation (10-20 ml) used in the studies had a molarity below 500 mmol/l; higher molarities increases the risk of skin side effects even if the injection site is changed every day. Despite application of the individual daily dose by two parallel running sc infusions via a twin pump, such limited skin tolerability is observed in particular with Neuroderm's ND0612 formulation (c.f. table 1.)

The tolerability of a levodopa subcutaneous infusion can further be improved by using customary solubilizing technologies in the infusion solution: Dissolving levodopa at a concentration necessary for the inventive method disclosed bears the risk of re-crystallization when the infusion solution mingles with the subcutaneous tissue fluid during infusion. Levodopa may then precipitate and local adverse effects may result. We have shown (in vitro) that by applying solubilization techniques, crystallization of levodopa is delayed, if the solution is in contact with the subcutaneous tissue fluid. Animal (pig) tests with the NeuroDerm formulation ND0612 showed black residues in skin specimens, suggesting local levodopa precipitations (Ramot et al. 2017)

The current invention has several clinical advantages:
1. Subcutaneous levodopa doses/day can be significantly reduced. With our invention a daily subcutaneous levodopa dose of 300 mg reaches constant plasma levels of around 1500 ng/ml. Prior art (NeuroDerm and AbbVie) infuses 720 or 680 mg/day and reaches only 800-1000 ng/ml levodopa plasma levels. According to our method, the increase of the daily subcutaneous dose to 600 mg/day levodopa generates levodopa plasma levels of around 3000 ng/ml which is enough to even treat late state Parkinson patients.
2. Subcutaneous infusion of levodopa generates constant levodopa plasma levels allowing Continuous Dopaminergic Stimulation at all individually needed plasma levels. Also, the invented treatment regimen markedly decreases interindividual and day to day intraindividual scatter in levodopa plasma levels because only the individual levodopa clearance rate defines the degree of plasma level variations. No erratic gastric emptying or other enteral disturbances known to be part of the disease will affect levodopa plasma levels.
3. Reduction of the subcutaneous levodopa dose significantly improves skin compatibility. Omitting the skin irritant carbidopa from the formulation, the compatibility of the subcutaneous infusion is further improved.
4. The effective and continuous inhibition of peripheral AADC entirely prevents the cumbersome peripheral dopaminergic side effect (e.g., nausea, cardiovascular effects), frequently observed in classical therapy of M. Parkinson.
5. Abandoning the present treatment paradigm calling for a fixed ratio of levodopa and an AADC-Inhibitor greatly simplifies the Parkinson treatment. Based on our effective inhibition of levodopa-metabolism by oral medication of the inhibitors, the subcutaneous levodopa dose can be easily modified according to individual needs, as required for the treatment of individually differing severity grades of the disease. Versatile pumps allow dosing according to daily needs, like night lowering of dose/h, morning dose, and additional bolus injections during the day.
6. Oral Add-On therapy of levodopa, if needed at all, is greatly facilitated: The underlying high levodopa plasma level of e.g., 1500 ng/ml established by the subcutaneously applied levodopa minimizes any fluctuations caused by an add-on Modified Release levodopa formulation. In addition, the increased half-life of levodopa (t1/2 = 3-4h) resulting from the inhibition of peripheral metabolism will additionally smoothen the amplitudes caused by any oral ad-on application of levodopa.
7. A further embodiment of the uniform application of low levodopa comprises the oral application of an oral Modified Release form of levodopa. Since the half-life of levodopa is tripled by the first part of the invention, a Modified Release form of levodopa leads to even stronger Continuous Dopaminergic Stimulation because any absorption problems from erratic gastric emptying of the PD patient is counteracted by the longer half-life of systemic levodopa.
8. As an important benefit for quality of life, the proposed combination regimen will allow most PD patients to completely spare the pump infusion overnight without losing adequate dopaminergic stimulation. Owing to the triple fold increase of the levodopa half-life, drug levels will fall rather slowly and provide sufficiently strong dopaminergic effect until the next morning dose, so that night time rigidity is improved.
9. Continuous Dopaminergic Stimulation is considered the optimal Parkinson therapy also for the purpose of preventing or postponing development of motor complications in the earlier state of the disease. Our invention may also be used for this purpose, because of its low local and systemic side effects.
10. There is growing evidence of a clear dose-dependent association between levodopa use and occurrence of peripheral neuropathy in PD patients. Our invention achieves identical plasma levodopa levels with only one third of the levodopa dose/day as required by any other currently available levodopa infusion therapy, and thus reduces the risk of peripheral neuropathies significantly.

### Examples

The current invention is further illustrated by the following examples which are not meant to be limiting.

### A) Materials and Methods:

### Example MM1:

### Production of a Modified Release multiparticle Formulation with 300 mg Carbidopa for oral application and rapid dissolution in the intestine

**A: Pellet manufacture:** In a first step 300 g of S-(-)-Carbidopa Monohydrate (100 - 150 U.S. mesh, LKT Lab.,Inc.) was mixed with 50 g α-Lactosemonohydrate (e.g. Granulac 200, Meggle) and 150 g Microcristalline Cellulose (Avicel PH 101, BASF) in a suited powder-mixer (Bohle) and then transferred to a dosing device of a twin screw extruder (e.g. 27GL-28D, Leistritz). The powdermix was extruded under wet granulation (aqua dem.) conditions to a wet extrudate and subsequently spheronized (e.g. RM 300, Schlüter) to a wet pellet mass. The wet pellet mass was dried at room temperature to a moisture content of 3.5 to 5% and then screened to a particle size of 0.8 to 1.2 mm.
**B: Coating:** 300 g of the dry pellets according to A are transferred in suited bottom spray coating device (fluidized bed, Glatt with Wurster processing device) and coated with an enteric coating polymer solution (e.g., EUDRAGIT FL 30D-55, aqueous dispersion, Evonik) up to a coating weight of 5% (after drying).
**C: Capsule Filling:** A gelatin hard capsule (size 0) was filled with 175 mg of uncoated pellets according to A and in addition with 350 mg of enteric coated pellets according to B giving a carbidopa Modified Release formulation with 1/3 of the total dose of 300 mg carbidopa as immediate release dose and 2/3 as Modified Release dose.
**D: Dissolution testing:** Testing of the carbidopa Modified Release formulation was conducted according to USP XXIV, Apparatus 1, Method A: Acid Stage: 2h at 37°C in 750 ml of 0.1N HCl, Buffer Stage by adding of 250 ml of 0.2 M tribasic sodium phosphate solution equilibrated to 37°C giving pH 6.8. Due to the pH-dependent solubility of Carbidopa and to safeguard sink conditions at any time point of the dissolution testing the capsules for the test were filled with a fraction of pellets according to A corresponding to a dose of 25 mg carbidopa, and in addition with a fraction of pellets according to B corresponding to a dose of 5mg carbidopa. The concentration of benserazide in the dissolution medium was determined by a validated HPLC method with UV detection.

The test capsules show after 30 minutes of starting the Acid Stage a mean dissolution rate (fraction dissolved) of 70 to 80% of the label claim of the fraction of pellets according to A (=25 mg carbidopa) and 30 minutes after starting the Buffer Stage a dissolution rate of 10-20% and 70-80% after 120 minutes of the label claim of the pellet fraction according to B (=5 mg carbidopa).

### Example MM2: Production of a Modified Release multiparticle Formulation with 300 mg Carbidopa for oral application and slow dissolution in the intestine.

**A: Pellet manufacture:** In a first step 300 g of S-(-)-Carbidopa Monohydrate (100 - 150 U.S. mesh, LKT Lab., Inc.) was mixed with 50 g α-Lactosemonohydrate (e.g., Granulac 200, Meggle) and 150 g Microcristalline Cellulose (Avicel PH 101, BASF) in a suited powder-mixer (Bohle) and then transferred to a dosing device of a twin screw extruder (e.g., 27GL-28D, Leistritz). The powder mix was extruded under wet granulation (aqua dem.) conditions to a wet extrudate and subsequently spheronized (e.g., RM 300, Schlüter) to a wet pellet mass. The wet pellet mass was dried at room temperature to a moisture content of 3.5 to 5% and then screened to a particle size of 0.8 to 1.2 mm.
**B: Coating:** 300 g of the dry pellets according to A are transferred in suited bottom spray coating device (fluidized bed, Glatt with Wurster processing device) and coated with an enteric coating polymer solution (e.g., EUDRAGIT FS 30 D, aqueous dispersion, Evonik) up to a coating weight of 5% (after drying).
**C: Capsule Filling:** A gelatin hard capsule (size 0) is filled with 175 mg of pellets according to A and in addition with 350 mg of enteric coated pellets according to B giving a Carbidopa Modified Release formulation with 1/3 of the total dose of Carbidopa as immediate release fraction and 2/3 as modified release fraction.
**D: Dissolution testing:** Testing of the carbidopa Modified Release formulation was conducted according to USP XXIV, Apparatus 1 (basket method), Method A: Acid Stage: 2h at 37°C in 750 ml of 0.1N HCl, Buffer Stage with adding of 250 ml of 0.2 M tribasic sodium phosphate solution equilibrated to 37°C giving pH 6.8. Due to the pH-dependent solubility of carbidopa and to safeguard sink conditions at any time point of the dissolution testing the capsules for the test were filled with a fraction of pellets according to A corresponding to a dose of 25 mg carbidopa and in addition with a fraction of pellets according to B corresponding to a dose of 5mg carbidopa. The concentration of carbidopa in the dissolution medium was determined by a validated HPLC method with UV detection.

The test capsules show 30 minutes after starting of the Acid Stage a mean dissolution rate (fraction dissolved) of 70 to 80% of the label claim of the fraction of pellets according to A (=25 mg carbidopa test dose) and 30 minutes after starting the Buffer Stage a mean dissolution rate of 10-20% and 70-80% after 300 minutes of the label claim of the pellet fraction according to B (=5 mg carbidopa test-dose).

### Example MM3: Production of a Modified Release Matrix Formulation with 300 mg Benserazide (as Benserazide × HCl) for oral application

**A: Powder blend manufacture:** In a first step 342 g of Benserazide × HCl drug substance (60 - 100 U.S. mesh, Sigma Aldrich) was mixed with 355 g Hydroxypropylmethylcellulose (HPMC, e.g., RetaLac, Meggle) in a suited Turbula mixer (Bachhoven, Basel) for 15 minutes. 3 g Mg stearate was added to the blend and mixed for another 1 minute to give the final composition of the tablet mass ready for direct compression.
**B: Tableting:** A single-punch tableting machine (Korsch, Berlin) equipped with flat-faces punches and suited diameter (e.g., 11.3 mm) was used to produce tablets with 700 mg total weight, hardness of 50-60N and 3.1-3.3 mm tablet height
**C: Dissolution testing:** Testing of the benserazide Modified Release formulation was conducted according to USP XXIV, Apparatus 2 (paddle method, 50 RPM), with 900ml of phosphate buffer solution (pH 6.8) equilibrated to 37°C. Benserazide is a highly water-soluble drug substance, sink conditions are given under the conditions selected. The concentration of benserazide in the dissolution medium was determined by a validated HPLC method with UV detection.

The test tablets show a mean dissolution rate (fraction dissolved) of 20-30 % dissolved of the label claim after 30 minutes of starting the dissolution test, 50-60% after 120 minutes and 80 - 90% after 300 minutes after starting the test procedure.

### Example MM4: Production of a Modified Release multiparticle Formulation with 300 mg Benserazide (as Benserazide × HCl) for oral application and slow dissolution in the intestine.

**A: Pellet manufacture:** In a first step 342 g of Benserazide × HCl (60 - 100 U.S. mesh, Sigma Aldrich) was mixed with 35 g α-Lactosemonohydrate (e.g., Granulac 200, Meggle) and 123 g Microcristalline Cellulose (Avicel PH 101, BASF) in a suited powder-mixer (Bohle) and then transferred to a dosing device of a twin screw extruder (e.g. 27GL-28D, Leistritz). The powder mix was extruded under wet granulation (aqua dem.) conditions to a wet extrudate and subsequently spheronized (e.g., RM 300, Schlüter) to a wet pellet mass. The wet pellet mass was dried at room temperature to a moisture content of 3.5 to 5% and then the pellets are screened to a particle size of 0.8 to 1.2 mm.
**B: Coating:** 300 g of the dry pellets according to A are transferred in suited bottom spray coating device (fluidized bed, Glatt with Wurster processing device) and coated with an enteric coating polymer solution (e.g., EUDRAGIT FS 30 D, aqueous dispersion, Evonik) up to a coating weight of 5% (after drying).
**C: Capsule Filling:** A gelatin hard capsule (size 0) is filled with 175 mg of uncoated pellets according to A and in addition with 350 mg of enteric coated pellets according to B giving a benserazide Modified Release hard capsule formulation with 1/3 of the total dose of benserazide as immediate release fraction and 2/3 as Modified Release fraction.
**D: Dissolution testing:** Testing of the benserazide Modified Release formulation was conducted according to USP XXIV, Apparatus 1 (basket method, 100 RPM), Method A: Acid Stage: 2h at 37°C in 750 ml of 0.1N HCl and Buffer Stage by adding of 250 ml of 0.2 M tribasic sodium phosphate solution equilibrated to 37°C giving pH 6.8. Benserazide is a highly water-soluble drug substance, sink conditions are given under the conditions selected. The concentration of benserazide in the dissolution medium was determined by a validated HPLC method with UV detection

The test capsules show a mean dissolution rate (fraction dissolved) of 70 to 80% of the label claim of the fraction of pellets according to A (=175 mg benserazide test dose) after 30 minutes of starting the Acid Stage and 30 minutes after starting the Buffer Stage a mean dissolution rate of 10-20% and 80 -90% after 300 minutes of the label claim of the pellet fraction according to B (=350 mg benserazide test-dose).

### Example MM5: Production of a gastro-retentive two-layer Matrix Formulation with 300 mg Benserazide (as Benserazide × HCl) for oral application and slow release in the Stomach

**A: Powder blend gas generating layer:** In a first step 120 g Polyoxyethylen (Polyox, WSR 303, Dupont), 20 g HPMC K4M (Methocel K4M, Dupont), 35 g Calcium carbonate and 25 g Sodium bicarbonate was mixed in a suited Turbula mixer (Bachhoven, Basel) for 15 minutes. Then 0.2 Mg Stearate was added to the blend and mixed for another 1 minute to give the final blend of the gas generating layer ready for direct compression.
**B: Powder blend slow-release drug containing Layer:** In a second step 75 g of Polyoxyethylen (Polyox, WSR 303, Dupont), 342 g of Benserazide × HCl drug substance (60 - 100 U.S. mesh, Sigma Aldrich), 58 g Lactose monohydrate (Meggle) was mixed in a suited Turbula mixer (Bachhoven, Basel) for 15 minutes. Then 0.5 g Mg Stearate was added to the blend and mixed for another 1 minute to give the final composition of the slow release drug containing layer ready for direct compression.
**C. Tableting:** A single-punch tableting machine (Korsch, Berlin) equipped with flat-faces punches and suited diameter (e.g., 11.3 mm) was used to produce tablets with 675 mg total weight, by stepwise transferring the powder blend of each layer and compressing the first layer by up to 500 MPa and after transferring the second layer the content was compressed by up to 1000 MPa for a compression cycle of about 30 seconds. Tablets show swelling after introduction into the dissolution medium and respective buoyancy for 6-8 hours.
**D: Dissolution testing:** Testing of the benserazide formulation was conducted according to USP XXIV, Apparatus 2 (paddle method, 50 RPM), with 900ml of 0,2 N HCl equilibrated to 37°C. The concentration of benserazide in the dissolution medium was determined by a validated HPLC method with UV detection.

The tablets tested show after a lag time of about 30 minutes a mostly linear mean dissolution rate (fraction dissolved) of 15 to 20 % per hour of the label claim.

### Example MM6: Production of a sterile ready to use solution with levodopa for sc infusion

7.5 g of Levodopa is suspended in a solution containing 3 g Acid ascorbicum and 30 g Arginine base dissolved in 450 ml water for injection. After stirring at room temperature for about 15 minutes levodopa is completely dissolved. The solution is adjusted to 500 ml with water for injection, showing finally a pH value of 9.1. The resulting slightly yellow clear solution is then filtered by a membrane filter and then by a sterile filter (0.2 µm) under aseptic conditions. The solution is filled to e.g. 20 mL into injection vials. The vials are stored protected from light at controlled temperatures between 2 - 25 °C (most preferable between 2 - 8 °C). 20 mL of the sterile solution is filled through the filling septum into the reservoir of an external infusion pump (Canè pump). Following activation of the pump the infusion rate is set to 0,84 ml/hour and the system delivers continuously 12.5 mg/h levodopa (=corresponding to 300 mg Levodopa for about 24 hours before a refill of the pump as described is necessary.

### Example MM7: Production of a sterile Lyophilisate with levodopa (15 mg/ml) for sc Infusion after Reconstitution before Use

1500 mg of levodopa is suspended in a solution containing 600 mg Acid ascorbicum, 3000 mg Arginine × HCl and 1500 mg Mannitol dissolved in 90 ml water for injection. After stirring at room temperature for about 15 minutes 1,5 to 2 ml of 5N NaOH is stepwise added to dissolve levodopa completely. The solution is adjusted to 100 ml total volume with water for injection, showing finally a pH value of 9.3. The resulting slightly yellow clear solution is then filtered by a sterile filter (0.2 µm) and then filled to 10 g each in suitable vials. After sealing with a suitable plug, the solution is frozen at minus 40 - 50 °C and then dried in a vacuum with use of a suitable freeze-dryer, whereby in a vial, a dry cake is produced from the formulation components. Then, the vials are sealed. In this way, a batch is produced with 10 vials (theoretical yield) with a single dose of 150 mg of levodopa. The lyophilisate thus obtained can be reconstituted with, e.g., water for injection (10 mL) in the vial and produces a solution that is appropriate for use for sc infusion for immediate use, whereby the composition of the solution with the selected adjuvants produces adequate stability under conditions of use of at least 36 hours

### Example MM8: Production of a gastro-retentive two-layer Matrix Formulation with 300 mg Carbidopa for oral application and slow release in the Stomach

**A: Powder blend gas generating layer:** In a first step 120 g Polyoxyethylen (Polyox, WSR 303, Dupont), 20 g HPMC K4M (Methocel K4M, Dupont), 35 g Calcium carbonate and 25 g Sodium bicarbonate was mixed in a suited Turbula mixer (Bachhoven, Basel) for 15 minutes. Then 0.2 Mg Stearate was added to the blend and mixed for another 1 minute to give the final blend of the gas generating layer ready for direct compression.
**B: Powder blend slow release drug containing Layer:** In a second step 75 g of Polyoxyethylen (Polyox, WSR 303, Dupont), 300 g of S-(-)- Carbidopa Monohydrate drug substance (100 - 150 U.S. mesh, IKT Lab., Inc.), 50 g Lactose monohydrate (Meggle) was mixed in a suited Turbula mixer (Bachhoven, Basel) for 15 minutes. Then 0.5 g Mg Stearate was added to the blend and mixed for another 1 minute to give the final composition of the slow release drug containing layer ready for direct compression.
**C. Tableting:** A single-punch tableting machine (Korsch, Berlin) equipped with flat-faces punches and suited diameter (e.g., 11.3 mm) was used to produce tablets with 625 mg total weight, by stepwise transferring the powder blend of each layer and compressing the first layer by up to 500 MPa and after transferring the second layer the content was compressed by up to 1000 MPa for a compression cycle of about 30 seconds. Tablets show swelling after introduction into the dissolution medium and respective buoyancy for 6-8 hours.
**D: Dissolution testing:** Testing of the carbidopa formulation was conducted according to USP XXIV, Apparatus 4 (flow-through cell), with a flow rate of 16 ml/min (with pulsation of 120±10 pulses per minute) of 0,2 N HCl equilibrated to 37°C as dissolution medium. At 30 minutes after starting the test and thereafter at hourly time points up to 8 hours a fraction of 10 ml of the eluate was collected. The concentration of carbidopa in the respective fraction was determined by a validated HPLC method with UV detection.

The tablets tested show after a lag time of about 30 minutes a mostly linear mean dissolution rate (fraction dissolved) of 15 to 20 % per hour of the label claim.

### Example MM9: Production of a sterile ready to use solution with levodopa for sc infusion

750 mg of levodopa is suspended in a solution containing 300 mg Acid ascorbicum and 3000 mg Arginine base dissolved in 45 ml water for injection. After stirring at room temperature for about 15 minutes levodopa is completely dissolved. The solution is adjusted to 50 ml with water for injection, showing finally a pH value of 9.1. The resulting slightly yellow clear solution is then filtered by a membrane filter and then by a sterile filter (0.2 µm) under aseptic conditions. The solution is filled to 10 mL into injection vials. The vials are stored protected from light at controlled temperatures between 2 - 25 °C (most preferable between 2 - 8 °C). 20 mL of the sterile solution is filled through the filling septum into the reservoir of an external infusion pump (Canè pump). Following activation of the pump the infusion rate is set to 0,84 ml/hour and the system delivers continuously 12.5 mg/h levodopa (=corresponding to 300 mg levodopa for about 24 hours before a refill of the pump as described is necessary.

### Example MM10: Production of a sterile Lyophilisate with Levodopa (15 mg/ml) for sc Infusion after Reconstitution before Use

1500 mg of levodopa is suspended in a solution containing 600 mg Acid ascorbicum, 3000 mg Arginine × HCl and 1500 mg Mannitol dissolved in 90 ml water for injection. After stirring at room temperature for about 15 minutes 1,5 to 2 ml of 5N NaOH is stepwise added to dissolve levodopa completely. The solution is adjusted to 100 ml total volume with water for injection, showing finally a pH value of 9.3. The resulting slightly yellow clear solution is then filtered by a sterile filter (0.2 µm) and then filled to 10 g each in suitable vials. After sealing with a suitable plug, the solution is frozen at minus 40 - 50 °C and then dried in a vacuum with use of a suitable freeze-dryer, whereby in a vial, a dry cake is produced from the formulation components. Then, the vials are sealed. In this way, a batch is produced with 10 vials (theoretical yield) with a single dose of 150 mg of levodopa. The lyophilizate thus obtained can be reconstituted with, e.g. water for injection (10 mL) in the vial and produces a solution that is appropriate for use for sc infusion for immediate use, whereby the composition of the solution with the selected adjuvants produces adequate stability under conditions of use of at least 36 hours.

### B) Clinical Studies:

In the following, ten clinical Phase I studies are presented. Altogether, 3-4 subjects received 44 treatments (12 times Pentadopa, 32 times levodopa) as either an intravenous bolus injection or as sc-infusions for 4, 16, or 24 hours. Total doses ranged from 13.6mg levodopa or 57.6mg Pentadopa (i.v.) to 300mg levodopa or 823mg Pentadopa (sc-infusion).

No peripheral side effects like nausea, vomiting, cardiovascular abnormalities or other side effects like headache, or singultus were observed during any of the treatments or thereafter.

### Example 1: The primary objective of the study was to determine the absolute bioavailability of levodopa (LD) from the penta-erythritol ester of levodopa (Pentadopa), and to measure levodopa plasma levels during and after subcutaneous infusion of Pentadopa combined with an oral inhibition of levodopa metabolism

Altogether four clinical Phase I studies were carried out. The study subjects (three male volunteers) were allocated to receive their study medication as described in Table 2a. Mean demographic data of the volunteers were: 72±3 yrs, 88±9 kg, 184±5 cm. Levodopa and 3-O-methy-levodopa (3-OMD) plasma levels were measured by use of a specific and validated HPLC method with mass detection.

**Table 2a: Design of Clinical Phase I Studies 1 to 4 with Pentadopa and Levodopa as Reference**

| **Study No** | **1** | | **2** | | **3** | **4** |
|---|---|---|---|---|---|---|
| **No of volunteers** | 3 | | 3 | | 3 | 3 |
| **No of study arms** | 2 | | 2 | | 1 | 1 |
| **Aim of study** | Abs. BA w/o COMT-I | | Abs. BA with COMT-I | | PK and Tolerability 4hours infusion | PK and Tolerability 24hours infusion |
| **Drug** | LD | Pentadopa | LD | Pentadopa | Pentadopa | Pentadopa |
| **Route of Admin.** | IV bolus | IV bolus | IV bolus | IV bolus | SC 4 hours | SC 24 hours |
| **Preparation** | AS 1 | AS 2 | AS 1 | AS 2 | AS 2 | AS 2 |
| **Total Dose (mg)*** | 25 | 57.5 | 13.6 | 109 | 137 | 823 |
| **mg Pentadopa/h** | n.a. | n.a. | n.a. | n.a. | 34.25 | 34.3 |
| **COMT-Inhibition** | No | no | 50mg Opicap. | 50mg Opicap. | 50 mg Opicapone | 50 mg Opicapone |
| | | | -24,-2h | -24,-2h | -12h, -2 h | -12h, -2 h, +4h, +14h |
| **AADC-Inhibition** | No | no | no | no | BZ 25mg/h | BZ 25mg/h |
| **Inhibition time** | n.a. | n.a. | n.a. | n.a. | -1h to +7h | -1h to +23h |
| **Analyte 1** | LD | LD | LD | LD | LD | LD |
| **Analyte 2** | 3OMD | 3OMD | 3OMD | 3OMD | 3OMD | 3OMD |

| | | | | | | |
|---|---|---|---|---|---|---|
| Explanations: Abs. BA = absolute Bioavailability; PK = Pharmacokinetics; COMT-I = Catechol-O-Methyl-Transferase Inhibition; Pentadopa = Pentaerythritol ester of levodopa; LD = levodopa; * = dose of Pentadopa as HCl salt; n.a. = not applicable; AADC-I = Aromatic Amino Acid Decarboxylase Inhibition; 3OMD = 3-O-methyl levodopa; BZ = benserazide; AS 1 = aqueous solution 1: water containing 10% (W/W) of 2-hydroxypropyl-β-cyclo-dextrine AS 2= 10mM phosphate buffer pH 4 | | | | | | |

Further study details: Two absolute bioavailability studies were carried out including one arm with intravenous levodopa as 100% reference and one arm with intravenous Pentadopa as test compound. In study 1, no inhibitor of drug metabolizing enzymes was co-administered. In study 2, 50mg opicapone was given before iv injection in order to estimate any 3-O-methylation of Pentadopa before ester cleavage.

Studies 3 and 4 investigated the levodopa plasma and 3OMD levels during and after the subcutaneous infusion of Pentadopa for 4 hours and 24 hours, respectively. Both studies were carried out with orally co-administered inhibitors of COMT (opicapone) and AADC (benserazide).

The results of the absolute bioavailability studies are given in Table 2. The levodopa levels generated by ester cleavage of intravenous Pentadopa were 35% of those of intravenous levodopa. When the COMT-inhibitor opicapone was co-administered, the absolute bioavailability increased to 49%, which indicate to a 3-O-methylation of some dose parts of the parent compound Pentadopa.

The iv-injection of levodopa in both the absolute bioavailability studies gave almost identical results. The total clearance was 9.8 to 10 ml/min/kg and the AUC without inhibition of metabolism accounted for 26.2ng*h/ml/mg levodopa.

**Table 2b: Pharmacokinetic Results of Studies 1 and 2;**

| **dose dependent parameters were calculated for a levodopa/levodopa-equivalent dose of 50 mg** | | | | |
|---|---|---|---|---|
| **Study No** | 1 | | 2 | |
| **No of volunteers** | 3 | | 3 | |
| **No of study arms** | 2 | | 2 | |
| **Aim of study** | Abs. BA w/o COMT-I | | Abs. BA with COMT-I | |
| **Drug** | LD | Pentadopa | LD | Pentadopa |
| **Route of Admin.** | IV bolus | IV bolus | IV bolus | IV bolus |
| **Preparation** | AS 1 | AS 2 | AS 1 | AS 2 |
| **Total Dose (mg)*** | 25 | 57.5 | 13.6 | 109 |
| | | | | |
| **t1/2 β (h)** | 1.70 | 1.12 | 1.26 | 1.21 |
| **MRT (h)** | 1.93 | 1.65 | 1.66 | 1.82 |
| **Cl (ml/min/kg)** | 9.80 | 22 | 10 | 15 |
| **AUC total (ng*h/ml)** | 1259 | 442 | 1363 | 680 |
| **BA (%)** | 100 | 35 | 100 | 49 |

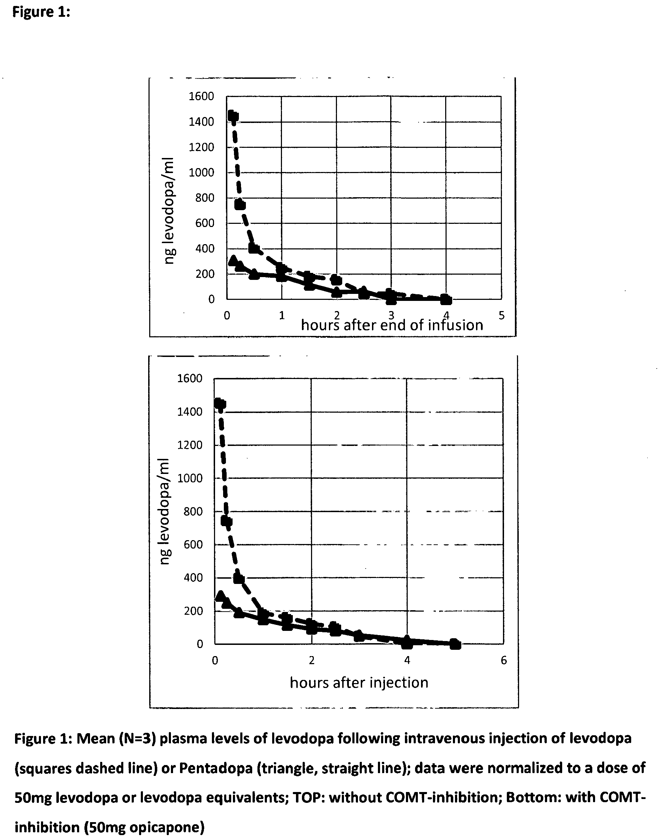

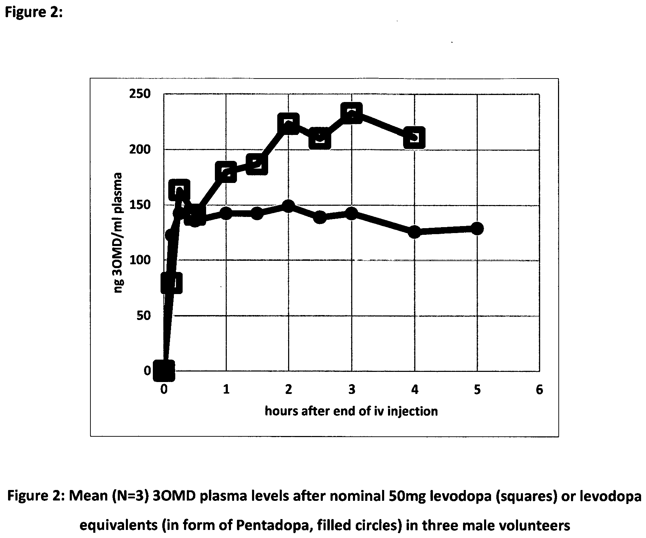

In Figure 1, levodopa plasma levels are shown, in Figure 2 respective 3OMD levels are displayed. 3OMD levels again show the incomplete ester cleavage of Pentadopa. In study 2, COMT was inhibited and almost no 3OMD levels were detectable.

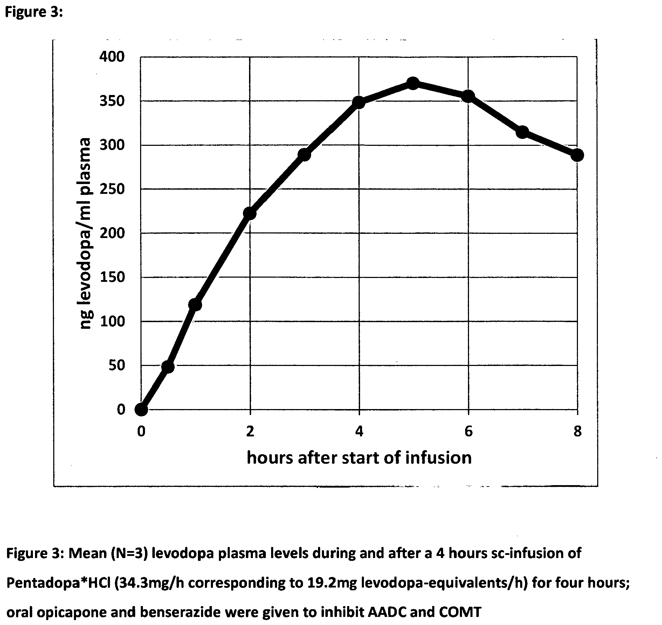

In study 3, Pentadopa was subcutaneously infused for 4 hours and levodopa plasma levels were determined. AADC and COMT were inhibited using benserazide (BZ) 25mg/h and 50mg opicapone, respectively. Table 3 gives the results and Figure 3 shows mean plasma levodopa levels. Throughout, 3OMD levels were low or undetectable.

Although only the truncated AUC₀₋₈ₕ was determined for (from the pro-drug released) levodopa, under the conditions of AADC- and COMT-inhibition the AUC/dose increased from 26.2ng*h/ml/mg (LD iv without AADC-inhibition, study 1) to 28.3 ng*h/ml/mg in study 3. This was surprising in respect to the about 50% ester cleavage. In other words, by inhibition of metabolism of levodopa the incomplete ester cleavage of Pentadopa is not only equalized but also surpassed when the total and not only truncated AUC is taken into account.

**Table 3: Pharmacokinetic (PK) Results of Study 3**

| **PK PARAMETERS of levodopa** | | | | | | |
|---|---|---|---|---|---|---|
| Subject | 1 | 2 | 3 | Mean | SD | CV (%) |
| AUC0-4 (ng*h/ml) | 919 | 686 | 828 | 811 | 117 | 14 |
| AUC0-8 (ng*h/ml) | 2619 | 1893 | 2001 | 2171 | 392 | 18 |
| Tmax (h) | 6 | 5 | 5 | 5.3 | 0.6 | 11 |
| Cmax (ng/ml) | 456 | 322 | 367 | 382 | 68 | 18 |
| linear increase (ng/ml/h) | 107 | 79 | 96 | 94 | 14 | 15 |

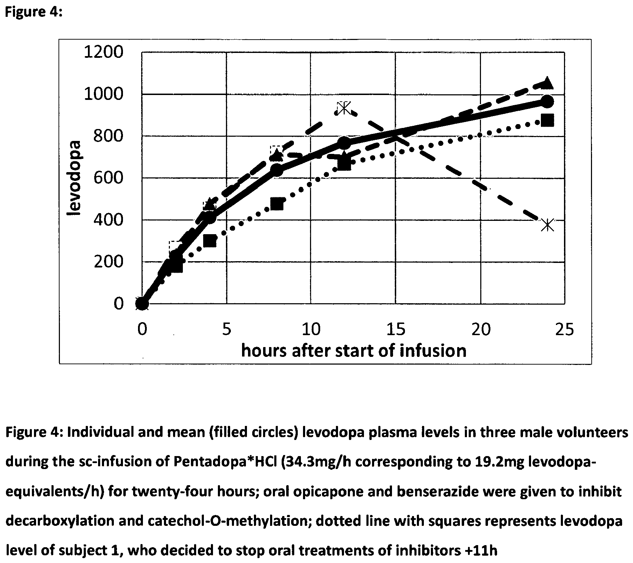

In study 4, Pentadopa was infused for 24 hours. AADC and COMT were inhibited by benserazide 25mg/h (-1h to +23h) and opicapone (50mg at -14, -2, +4, and +14 hours), respectively. Figure 4 gives the plasma level time courses.

The increases in levodopa plasma levels were similar in all three volunteers. Coefficients of variation of mean values were below 25%. In agreement with study 3, mean 4-hour value was about 400 ng/ml corresponding to a linear increase rate of roughly 100ng/ml/h. After the first 4 to 6 hours of infusion, a slower increase rate was observed. At the end of the 24 hours infusion, probably a steady-state was reached characterized by a levodopa plasma level of 1000 ng/ml (Figure 4). The levodopa equivalent dose to reach this steady-state condition was 461mg/d.

### Example 2:

### The primary objective of the study was to evaluate the dose dependent effects of continuous enteral administration of benserazide (BZ) on levodopa (LD) plasma levels (systemic availability) after sc-infusion of levodopa

Altogether three clinical Phase I studies were performed with the aim to investigate the dose dependency of oral benserazide on the levodopa-levels during a four hours sc-infusion of levodopa. Three male volunteers are allocated to the study medication as described in detail in Table 4. Levodopa plasma levels were either followed during infusion (both arms of study 7) or during infusion and 4 hours thereafter. Benserazide doses were 12.5, 25, and 50mg/h during the time of levodopa plasma level determinations.

Results are summarized in Table 5.

**Table 4: Details of Clinical Phase I studies 5 to 7 with Benserazide as AADC-Inhibitor**

| **Study No** | **5** | **6** | **7** | |
|---|---|---|---|---|
| **No of volunteers** | 3 | 3 | 3 | |
| **No of study arms** | 2* | 1 | 2 | |
| **Aim of study** | LD pharmacokinetics | LD pharmacokinetics | LD pharmacokinetics | |
| **Drug** | LD | LD | LD | |
| **Route of Admin.** | Sc | Sc | sc | |
| **Preparation** | AS 4 | AS 5 | AS 6 | |
| **Total Dose (mg)** | 50.4 in 4 hours | 50mg in 4 hours | 50mg in 4 hours | |
| **BZ (mg)/h)** | 12.5 | 25 | 25 | 50 |
| **Inhibition time** | -1 to+7 h | -1 to +7 h | -1 to+3h | -1to+3h |
| **COMT-Inhibition** | Opicapone 50mg | Opicapone 50mg | OC50mg | OC50mg |
| **Inhibition times** | -24h, -2h | -12h, -2h | -24h,-2h | -24h, - 2h |

| | | | | |
|---|---|---|---|---|
| *The second arm was investigating carbidopa and will be included in Example 3; AS 4 = 30mg LD, 6mg Ascorbic acid, 115mg Arginine per 1 ml water, pH 9.1 (titrated); AS 5 = 3mg LD, 0.15g 2-OH-propyl-β-cyclodextrine per 1ml water; AS 6 = 15mg LD, 6mg Ascorbic acid, 60 mg Arginine per 1ml water, pH 8.75 (titrated) OC = opicapone | | | | |

**Table 5: Pharmacokinetic Results of Clinical Phase I Studies 5 to 7 with Benserazide (BZ)**

| **Study** | **5** | **6** | **7** | |
|---|---|---|---|---|
| **Parameter** | **12.5mg BZ** | **25mg BZ** | **25mg BZ** | **50mg BZ** |
| **Cmax ng/ml** | 278±34 | 598±108 | 503±85 | 542±112 |
| **Tmax (h)** | 4±0 | 4.7±0.6 | 4.3±0.3 | 4.0±0 |
| **AUD 0-4 h** | 539±165 | 1150±356 | 932±220 | 1128±116 |
| **AUD total** | 1829±501 | 4015±815 | n.c. | n.c. |
| **CL _{(ml/min/kg)}** | 5.5±1.2 | 2.4±0.3 | n.c. | n.c. |
| **Increase ng/ml/h** | 69±15 | 80±12 | 116±33 | 131±26 |

The Pharmacokinetic parameters Cₘₐₓ, AUC₀₋₄ₕ, and plasma level increase/hour clearly increased proportional with the benserazide dose in studies 5 and 7. In study 6, the opicapone 50mg doses were given at -12 and -2 hours and thus, most probably a higher COMT-inhibition was reached. The effect of the more effective COMT-inhibition was about 20% increase of the AUC ₀₋₄ₕ of levodopa. A similar effect was seen by the increase of benserazide from 25mg/h to 50mg/h.

### Example 3

### The primary objective of the study was to evaluate the dose dependant effects of continuous enteral administration of carbidopa (CD) on levodopa plasma levels (systemic availability) after sc-infusion of levodopa

Three clinical Phase I studies were performed to investigate the dose dependant effects of carbidopa on levodopa plasma levels. Table 6 gives the study details.

In studies 5 and 8, levodopa was given by sc-infusion for four hours. The total dose was 50mg or 12.5mg/h. In study 9, the total levodopa dose was 300mg in 16 hours or 18.75mg/h. Due to an application failure in 2 subjects, that was noticed after 2 hours, the total observation time for all subject was reduced to 14 hours.

**Table 6: Details of Clinical Phase I Studies 5, 8, and 9 with Carbidopa as AADC-Inhibitor**

| **Study No** | **5** | **8** | | **9** |
|---|---|---|---|---|
| **No of volunteers** | 3 | 2 | | 3 |
| **No of study arms** | 2* | 2 | | 1 |
| **Aim of study** | LD pharmacokinetics | LD pharmacokinetics | | LD pharmacokinetics |
| **Drug** | LD | LD | | LD |
| **Route of Admin.** | Sc | Sc | | Sc |
| **Preparation** | AS 4 | AS 6 | | AS 6 |
| **Total Dose (mg)** | 50.4 in 4 hours | 50mg in 4 hours | | 300mg in 16 hours |
| **Dose (mg/h)** | 12.6 | 12.5 | | 18.75 |
| **CD (mg)/h** | 12.5 | 25 | 50 | 25 |
| **Inhibition time** | -1 to+7 h | -1to+7h | -1to+7h | -1 to +15 hours |
| **COMT-Inhibition** | Opicapone 50mg | OC 50mg | OC 50mg | OC 50mg |
| **Inhibition times** | -24h, -2h | -24h, -2h | -24h, -2h | every 6 hours from -26h to+10h |

| | | | | |
|---|---|---|---|---|
| *The second arm was investigating benserazide and was included in Example 2; AS 6 and AS 4: see footnote Table 4; OC = opicapone | | | | |

**Table 7: Pharmacokinetic Levodopa Results of Clinical Phase I Studies 5, 8, and 9 with Carbidopa**

| **Study** | **5** | **8** | | **9** |
|---|---|---|---|---|
| **Parameter** | **12.5mg CD** | **25mg CD** | **50mg CD** | **25mg CD** |
| **Cmax/C4h (ng/ml)** | 330±57 | 362±63 | 436±84 | 443±110* |
| **Tmax (h)** | 5±0 | 5±0 | 4.5±0.5 | n.a. |
| **AUC 0-4 h** | 677±12 | 729±91 | 902±130 | 962±169* |
| **AUC total** | 2096±122 | 2518±84 | 2969±236 | n.a. |
| **CL _{(ml/min/kg)}** | 4.6±0.3 | 3.7±0.2 | 3.1±0 | n.a. |
| **Increase ng/ml/h** | 84±5 | 81±14 | 96±21 | 113±1* |

| | | | | |
|---|---|---|---|---|
| * re-calculated for a dose of 12.5mg/h; n.a. = not applicable | | | | |

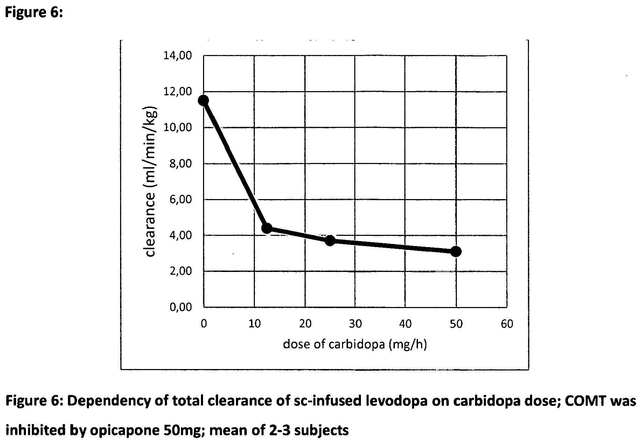

Comparing the studies with 4 hours sc-infusion, the Cₘₐₓ, AUD₀₋₄ₕ and AUD_{0-inf} values increased with increasing carbidopa dose and clearance rates decreased from 4.6ml/min/kg to 3.1ml/min/kg (Table 7, Figure 6). After recalculation to the same levodopa dose/hour, the mean AUD₀₋₄ₕ accounts for 962ng*h/ml which is 32% higher than in study 8. Most probably, this difference reflects the more effective COMT-inhibition achieved in study 9.

### Example 4

### The effect of two different modes of COMT-inhibition on the levodopa plasma level profiles during 16 or 24 hours sc-infusions of levodopa (carbidopa (CD) co-administration: 25 mg/h)

Two studies were performed to investigate the role of COMT-inhibition (Table 8). In study 10, opicapone (OC) 50mg was administered 3 times at an interval of 24hours: -26hours, -2hours, +22 hours with 0 = start of infusion. In study 9, opicapone (OC) 50mg was given every 6 hours starting at -26 and then at -20h, -14h, -8h, -2h, +4h, +10h. This treatment was chosen to mimic the clinical situation characterised by an accumulation of inhibitory potency over about 10 days of daily treatment with 50mg opicapone.

**Table 8: Details of clinical Phase I studies 9 and 10**

| **Study No** | **9** | **10** |
|---|---|---|
| **No of volunteers** | 3 | 4 |
| **No of study arms** | 1 | 1 |
| **Aim of study** | LD pharmacokinetics 16h | LD pharmacokinetics 30 hours |
| **Drug** | LD | LD |
| **Route of Admin.** | sc | sc |
| **Preparation** | AS 6 | AS 6 |
| **Total Dose (mg)** | 300mg in 16 hours | 300mg in 24 hours |
| **Dose mg/h** | 18.75 | 12.5 |
| **CD (mg)/h** | 25 | 25 |
| **Inhibition time** | -1 to +15 hours | -1h to 24h |
| **COMT-Inhibition** | OC 50mg | OC 50mg |
| **Inhibition times** | every 6 hours from -26h to+10h | -24h, -2h, +22h |

In study 10, the mean total AUC was 16287ng*h/ml corresponding to 54.3ng*h/ml/mg levodopa. This number is 2.1fold higher than for non-inhibited levodopa (see Example 1) and one third lower than was expected from 4-hours study 6 (Example 2). The reason clearly was the insufficient inhibition of COMT in study 10. This is also reflected by the higher clearance of 4ml/min/kg as compared to study 6. The half-life of levodopa disposition was calculated to 4.1±0.7 hours (Table 10).

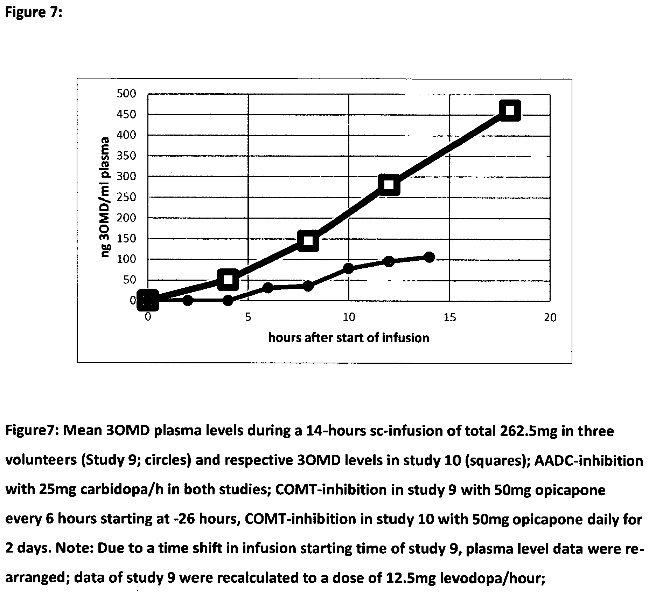

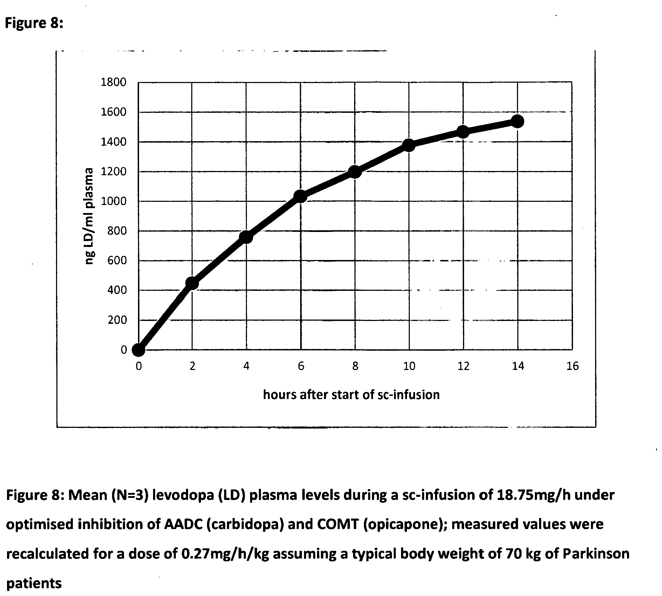

In study 9, the COMT-inhibition was mimicked in a probably more reliable way (Figure 7) to the clinical situation and the 300mg levodopa-dose was given within 16 hours. According to a defect between tube and pump, two subjects received treatment 2 hours later. Therefore, data of all three subjects were re-arranged to a total dose of 262.5 mg infused over 14 hours. Figure 8 gives the mean plasma levels of study 9, corrected for a typical body weight of PD patients of 70 kg.

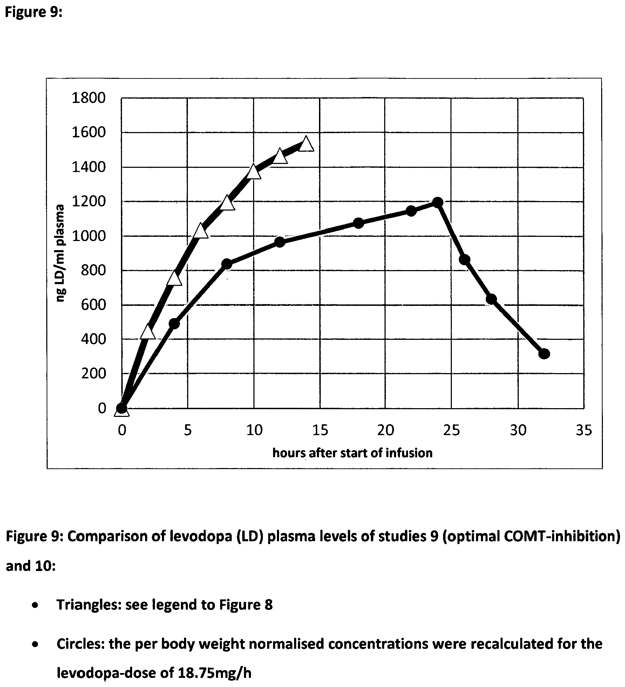

In order to combine both studies 9 and 10, Figure 9 was generated. Data from Figure 8 are combined for the mean levodopa profile of study 11, that was likewise normalised to a body weight of 70 kg. In addition, these data were re-calculated for the same infusion rate as in study 9: 18.75mg levodopa/h. The comparison clearly shows that in Parkinson's disease patients a steady state plasma level of 1200ng/ml will be achieved using an infusion rate of 18.75mg/h and a suboptimal COMT-inhibition. Under the same conditions but under an optimised COMT-inhibition the steady state plasma level will be around 1800 ng/ml. In most patients, reduction of infusion rates (day-night-morning) are needed for optimal control of clinical symptoms. This can easily be achieved by changes in infusion rates (ml/h).

**Table 10: Pharmacokinetic Levodopa Results of Clinical Phase I Study 10**

| **Subject/ Parameter** | **1** | **2** | **3** | **4** | **mean** | **sd** |
|---|---|---|---|---|---|---|
| **AUC total (ng*h/ml)** | 15166 | 11395 | 12686 | 25899 | 16287 | 5713 |
| **Cl (ml/min/kg)** | 3.97 | 4.48 | 4.75 | 2.93 | 4.0 | 0.7 |
| **T1/2 (h)** | 3.54 | 3.69 | 4.03 | 5.33 | 4.1 | 0.7 |

### Example 5:

### Simulation of carbidopa plasma levels in studies 9 and 10 and modeling of Modified Release carbidopa preparations in order to reach equivalent systemic inhibitor concentrations

In order to get plasma level concentrations of carbidopa under the hourly treatment schedule, a simulation was carried out on the basis of a single dose experiment published by Hsu et al. (Hsu A et al.: Comparison of the Pharmacokinetics of an Oral Extended-Release Capsule Formulation of carbidopa-levodopa (IPX066) With Immediate-Release carbidopa-levodopa (Sinemet1), Sustained-Release carbidopa-levodopa (Sinemet1 CR), and carbidopa-levodopa-entacapone (Stalevo1); The Journal of Clinical Pharmacology 2015, 55(9) 995-1003 © 2015 DOI: 10.1002/jcph.514).

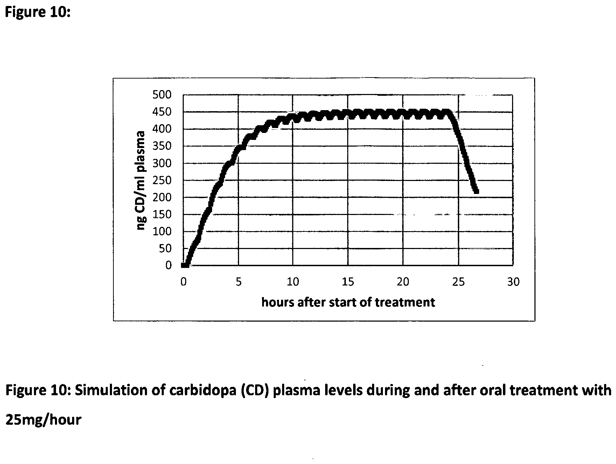

Concentrations were read out from Figure 1B for the immediate release formulation IR-CD25-LD100 and fitted by means of a 1-compartment body model (TopFit 2.0). Calculated micro-constants were used to simulate the hourly 25mg dose given in studies 9 or 10. Figure 10 gives the result. Steady-state conditions of 450ng/ml were reached after 10 to 15 hours.

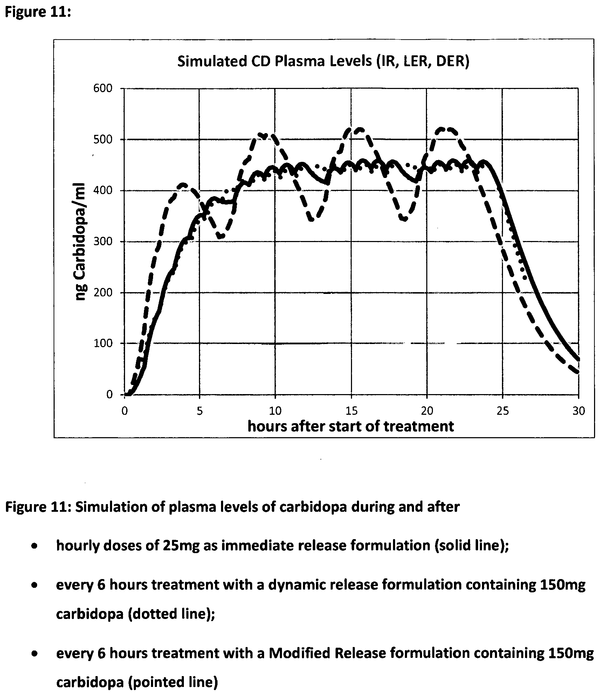

The simulation of carbidopa plasma levels of two different formulations (dynamic or linear release over 6 hours) clearly showed that a formulation with linear release of carbidopa is much better suited to mimic the immediate release formulation situation (Figure 11).

### Brief Description of the Drawings:

Figure 1 shows mean (N=3) plasma levels of levodopa following intravenous injection of levodopa (squares dashed line) or Pentadopa (triangle, straight line); data were normalized to a dose of 50mg levodopa or levodopa equivalents; Top: without COMT-inhibition; Bottom: with COMT-inhibition (50mg opicapone)
Figure 2 shows mean (N=3) OMD plasma levels after nominal 50mg levodopa (squares) or levodopa equivalents (in form of Pentadopa, filled circles) in three male volunteers
Figure 3 shows mean (N=3) levodopa plasma levels during and after a 4 hours sc-infusion of Pentadopa*HCl (34.3mg/h corresponding to 19.2mg levodopa-equivalents/h) for four hours; oral opicapone and benserazide were given to inhibit AADC and COMT
Figure 4 shows individual and mean (filled circles) levodopa plasma levels in three male volunteers during the sc-infusion of Pentadopa*HCl (34.3mg/h corresponding to 19.2mg levodopa-equivalents/h) for twenty-four hours; oral opicapone and benserazide were given to inhibit decarboxylation and catechol-O-methylation; dotted line represents levodopa level of subject 1, who decided to stop oral treatments of inhibitors +11h
Figure 5 shows increase of AUC₀₋₄ₕ of levodopa during a sc-infusion of 12.5mg levodopa/h in dependency of oral benserazide (BZ) (triangle) or carbidopa (CD) (crosses); doses were 12.5, 25, 50mg/h; COMT-inhibition by 50mg opicapone (-24, -2h); single points show levodopa AUC with 25mg benserazide/h but with 50mg opicapone at 6 hours distance (top bullet) or with 25 mg carbidopa but with 50mg opicapone at 12 hours distance (lower bullet)
Figure 6 shows the dependency of total clearance of sc-infused levodopa on carbidopa dose; COMT was inhibited by opicapone 50mg; mean of 2-3 subjects
Figure7 shows mean 3OMD plasma levels during a 14-hours sc-infusion of total 262.5mg in three volunteers (Study 9; circles) and respective 3OMD levels in study 10 (squares); AADC-inhibition with 25mg carbidopa/h in both studies; COMT-inhibition in study 9 with 50mg opicapone every 6 hours starting at -26 hours, COMT-inhibition in study 10 with 50mg opicapone daily for 2 days. Note: Due to a time shift in infusion starting time of study 9, plasma level data were re-arranged; data of study 9 were recalculated to a dose of 12.5mg levodopa/hour;
Figure 8 shows mean (N=3) levodopa plasma levels during a sc-infusion of 18.75mg/h under optimised inhibition of AADC (carbidopa) and COMT (opicapone); measured values were recalculated for a dose of 0.27mg/h/kg assuming a typical body weight of 70 kg of PD patients Figure 9 shows a comparison of levodopa plasma levels of studies 9 and 10:
   - Triangles: see legend to Figure 7
   - Squares: Levodopa levels measured in study 10 normalised to a body weight of 70kg
   - Circles: the per body weight normalised concentrations were recalculated for the levodopa -dose of 18.75mg/h
Figure 10 shows a simulation of carbidopa plasma levels during and after oral treatment with 25mg/hour
Figure 11 shows a simulation of plasma levels of carbidopa during and after
   - hourly doses of 25mg as immediate release formulation (Red);
   - every 6 hours treatment with a Modified Release formulation containing 150mg carbidopa (Blue);
   - every 6 hours treatment with a linear release formulation containing 150mg carbidopa (Green)

**Table 1:**

| Comparison of Abbvie-951, ND0612H, and Berlirem SC formulation: Molecular Burden, Daily levodopa doses and attainable levodopa plasma levels | | | | | | | | |
|---|---|---|---|---|---|---|---|---|
| | | Conc. mg/ml | Injection vol. ml | Molarity (mmol/l) | Total molarity (mmol/l) | Daily levodopa equivalent dose mg | Reached levodopa plasma level ng/ml | Efficiency Factor: levodopa plasma levels (ng/ml) reached by 100 mg sc levodopa/day |
| ABBV-951 | Foslevodopa | 240 | 4⁴ | 1056 | 1095 | 680 | ≈ 800 | 118 |
| pH 7.4 | Foscarbidopa | 12 | | 39 | | | | |
| 28 mg/h levodopa | | | | | | | | |
| ND0612H | levodopa | 60 | 2x6 =12⁴ | 304 | 912 | 720 | ≈ 800 | 111 |
| Two injection sites! | Carbidopa | 14 | | 61 | | | | |
| pH 9.5? | Arginine 1.8 molar¹ | | | 547 | | | | |
| 30 mg/h levodopa | | | | | | | | |
| | | | | | | | | |
| Berlirem A | levodopa ² | 15/30 | 20/10⁵ | 152³ | 425 | 300 | 1500 | 500 |
| pH 9.1 | | | | | | | | |
| | Aqueous buffer | | | 273³ | | | | |
| Berlirem B | | 30 | 2×10=20⁵ | | 425 | 600 | 3000 | 500 |
| Two injection sites! | | | | | | | | |
| 25 mg/h levodopa | | | | | | | | |
| • ¹ Neuroderm Patent WO2012/066538 Claim 2.: The pharmaceutically acceptable composition of claim 1, wherein the molar ratio of active components to the arginine is about 1:1.8 to about 1:3.5, or about 1:2.3. | | | | | | | | |
| • ²without (!) Carbidopa | | | | | | | | |
| • ³ Molarity calculated on the basis of 30mg/ml levodopa in aqueous buffer solution | | | | | | | | |
| • ⁴ Injected over 24 h | | | | | | | | |
| • ⁵To provide nocturnal dose lowering injected over 16 h | | | | | | | | |

### References

Nishikawa N, Iwaki H, Shiraishi T, Mukai Y, Takahashi Y, Murata M.
Female, aging, difference formulations of DCI, or lower body weight increases AUC4hr of levodopa in patients with Parkinson's disease. Parkinsonism Relat Disord. 2020 Jul;76:16-20. doi: 10.1016/j.parkreldis.2020.05.020. Epub 2020 Jun 2. PMID: 32554330
Iwaki, H; Noriko Nishikawa, Masahiro Nagai, Tomoaki Tsujii, Hayato Yabe, Madoka Kubo, Ichiro leiri, Masahiro Nomoto
Pharmacokinetics of levodopa/benserazide versus levodopa/carbidopa in healthy subjects and patients with Parkinson's disease. Neurology and Clinical Neuroscience 3 (2015) 68-73
Greenacre JK, Coxon A, Petrie A, Reid JL.
Comparison of levodopa with carbidopa or benserazide in parkinsonism. Lancet. 1976 Aug 21;2(7982):381-4. doi: 10.1016/s0140-6736(76)92403-x. PMID: 73849
Brod LS, Aldred JL, NuttJG.
Are high doses of carbidopa a concern? A randomized, clinical trial in Parkinson's disease.
Mov Disord. 2012 May;27(6):750-3. doi: 10.1002/mds.24998. Epub 2012 Apr 16. PMID: 22508376
Dingemanse J, Kleinbloesem CH, Zürcher G, Wood ND, Crevoisier C.
Pharmacodynamics of benserazide assessed by its effects on endogenous and exogenous levodopa pharmacokinetics. Br J Clin Pharmacol. 1997 Jul;44(1):41-8. doi: 10.1046/j.1365-2125.1997.00610.x. PMID: 9241095
Männistö PT, Ulmanen I, Lundström K, Taskinen J, Tenhunen J, Tilgmann C, Kaakkola S. Characteristics of catechol O-methyl-transferase (COMT) and properties of selective COMT inhibitors.
Prog Drug Res. 1992;39:291-350. doi: 10.1007/978-3-0348-7144-0_9. PMID: 1475365
Shoulson I, Glaubiger GA, Chase TN.
On-off response. Clinical and biochemical correlations during oral and intravenous levodopa administration in parkinsonian patients. Neurology. 1975 Dec;25(12):1144-8. doi: 10.1212/wnl.25.12.1144. PMID: 812004
Quinn N, Marsden CD, Parkes JD.
Complicated response fluctuations in Parkinson's disease: response to intravenous infusion of levodopa. Lancet. 1982 Aug 21;2(8295):412-5. doi: 10.1016/s0140-6736(82)90442-1. PMID: 6124807
Nutt JG, Woodward WR, Hammerstad JP, Carter JH, Anderson JL.
The "on-off" phenomenon in Parkinson's disease. Relation to levodopa absorption and transport. N Engl J Med. 1984 Feb 23;310(8):483-8. doi: 10.1056/NEJM198402233100802. PMID: 6694694
Nyholm D, Odin P, Johansson A, Chatamra K, Locke C, Dutta S, Othman AA. Pharmacokinetics of levodopa, carbidopa, and 3-O-methyldopa following 16-hour jejunal infusion of levodopa-carbidopa intestinal gel in advanced Parkinson's disease patients. AAPS J. 2013 Apr;15(2):316-23. doi: 10.1208/s12248-012-9439-1. Epub 2012 Dec 11. PMID: 23229334
Y. Caraco, S. Oren, O. Yacoby-Zeevi, P. LeWitt, N. Giladi
ND0612, A NOVEL FORMULATION OF LEVODOPA/CARBIDOPA FOR CONTINUOUS, SUBCUTANEOUS ADMINISTRATION, ACHIEVES STEADY-STATE LEVODOPA PLASMA CONCENTRATIONS IN PARKINSON'S DISEASE PATIENTS (Poster 17th International Congress of Parkinson's Disease and Movement Disorders, June 2013, Sydney, Australia - Abstract number: LBA26)
Romagnolo A, Merola A, Artusi CA, Rizzone MG, Zibetti M, Lopiano L.
Levodopa-Induced Neuropathy: A Systematic Review.
Mov Disord Clin Pract. 2018 Nov 8;6(2):96-103. doi: 10.1002/mdc3.12688. eCollection 2019 Feb. PMID: 30838307
Matthew Rosebraugh, Philip Kym, Wei Liu, Maurizio Facheris, Janet Benesh
A Novel Levodopa/Carbidopa Prodrug (ABBV-951) 24-Hour Continuous Subcutaneous Infusion Treatment for Parkinson's Disease (P3.8-037). Neurology: April 09, 2019; 92 (15 Supplement) May 7, 2019
LeWitt PA.
Levodopa therapy for Parkinson's disease: Pharmacokinetics and pharmacodynamics.
Mov Disord. 2015 Jan;30(1):64-72. doi: 10.1002/mds.26082. Epub 2014 Dec 1. PMID: 25449210
M. Stranz and E.S. Kastango
A REVIEW of pH AND OSMOLARITY
International Journal of Pharmaceutical Compounding 6:217-220 (2002)
US 4,409,233
Trukuda, K., Kato, W. (1979)
Highly Concentrated Preparations of DOP Compounds
WO 2012/066538 A1
Yacoby-Zeevi, O., Nemas, M. (2012)
CONTINUOUS ADMINISTRATION OF L-DOPA, DOPA DECARBOXYLASE INHIBITORS, CATECHOL-O-METHYLTRANSFERASE INHIBITORS AND COMPOSITIONS FOR SAME
Hsu A et al.: Comparison of the Pharmacokinetics of an Oral Extended-Release Capsule Formulation of Carbidopa-L-Dopa (IPX066) With Immediate-Release Carbidopa-L-Dopa (Sinemet1), Sustained-Release Carbidopa-L-Dopa (Sinemet1 CR), and Carbidopa-Levodopa-Entacapone (Stalevo1); The Journal of Clinical Pharmacology 2015, 55(9) 995-1003 © 2015 DOI: 10.1002/jcph.514).
ANDERE (nicht zitiert):
   US 2021/0085635 A1 (Orion)
   ROURU, Juha; Mikko KUOPPAMÄKI, Raisio ( FI ) ; Juha ELLMEN , Turku ( FI ) ; Pekka MÄNNISTÖ, USE OF LEVODOPA, CARBIDOPA AND ENTACAPONE FOR TREATING PARKINSON'S DISEASE
Trenkwalder, C. MD, Mikko Kuoppamäki, MD, PhD, Mikko Vahteristo, MSc, Thomas Müller, MD, PhD, and Juha Ellm'en, PhD
Increased dose of carbidopa with levodopa and entacapone improves "off" time in a randomized trial
Neurology® 2019;92:e1487-e1496. doi:10.1212/WNL.0000000000007173
US 2016/0362431 A1 (AbbVie)
CARDINAL-DAVID, B. et al.
CARBIDOPA AND L-DOPA PRODRUGS AND METHODS OF USE
EP 3838263 A1 (Dizlin)

## Claims

1. A kit of pharmaceutical preparations for use in the treatment of Parkinson's Disease or Restless Legs Syndrome, containing
a) an AADC-inhibitor selected from benserazide or carbidopa,
wherein the AADC-inhibitor is applied in a daily dose of 300-1200mg
wherein said AADC-inhibitor is provided as an oral Modified Release formulation,
wherein the oral Modified Release formulation of the AADC-inhibitor is constantly releasing the AADC-inhibitor in the gastrointestinal tract in an amount of 12.5-50 mg/h over at least 6 hours, and
wherein the oral Modified Release formulation of the AADC-inhibitor is applied 2-4 times a day
b) a liquid formulation of levodopa or levodopa ester,
wherein the liquid formulation contains levodopa or levodopa ester in a molarity of 100-500 mmol/l
wherein the liquid formulation is subcutaneously infused
wherein the daily dose of levodopa or levodopa equivalent is 100-900mg
c) optionally, a pharmaceutical preparation of a COMT-inhibitor in which the COMT-inhibitor is 25-75 mg Opicapone.

2. A kit for use according to claim 1, in which the oral Modified Release formulation of the AADC-inhibitor is provided as a tablet or capsule each containing 200-400mg benserazide or carbidopa.

3. A kit for use according to claim 1, in which the oral Modified Release formulation of an AADC-inhibitor is provided as a tablet or capsule each containing 200-400mg benserazide.

4. A kit for use according to at least one of claims 1-3, the levodopa ester is the penta-erythritol ester of levodopa.

## Patentansprüche

1. Kit von pharmazeutischen Zubereitungen zur Verwendung bei der Behandlung der Parkinson-Krankheit oder des Restless-Legs-Syndroms, enthaltend
a) einen AADC-Inhibitor, ausgewählt aus Benserazid oder Carbidopa,
wobei der AADC-Hemmer in einer Tagesdosis von 300-1200 mg verabreicht wird wobei der AADC-Inhibitor als orale Formulierung mit modifizierter Freisetzung bereitgestellt wird,
wobei die orale Formulierung mit modifizierter Freisetzung des AADC-Inhibitors den AADC-Inhibitor im Gastrointestinaltrakt in einer Menge von 12,5-50 mg/h über mindestens 6 Stunden kontinuierlich freisetzt, und
wobei die orale Formulierung mit modifizierter Freisetzung des AADC-Inhibitors 2-4 Mal pro Tag angewendet wird
b) eine Flüssigformulierung von Levodopa oder Levodopa-Ester,
wobei die flüssige Formulierung Levodopa oder Levodopa-Ester in einer Molarität von 100-500 mmol/l enthält
wobei die Flüssigformulierung subkutan infundiert wird
wobei die Tagesdosis an Levodopa oder Levodopa-Äquivalent 100-900 mg beträgt
c) gegebenenfalls eine pharmazeutische Zubereitung eines COMT-Inhibitors, in der der COMT-Inhibitor 25-75 mg Opicapon ist.

2. Kit zur Verwendung nach Anspruch 1, bei dem die orale modifizierte Freisetzungsformulierung des AADC-Inhibitors als Tablette oder Kapsel bereitgestellt wird, die jeweils 200-400 mg Benserazid oder Carbidopa enthält.

3. Kit zur Verwendung nach Anspruch 1, in dem die orale modifizierte Freisetzungsformulierung eines AADC-Inhibitors als Tablette oder Kapsel bereitgestellt wird, die jeweils 200-400 mg Benserazid enthält.

4. Kit zur Verwendung nach mindestens einem der Ansprüche 1 bis 3, wobei der Levodopa-Ester der Penta-Erythritol-Ester von Levodopa ist.

## Revendications

1. Kit de préparations pharmaceutiques à utiliser dans le traitement de la maladie de Parkinson ou du syndrome des jambes sans repos, contenant
a) un inhibiteur de l'AADC choisi parmi le bensérazide ou la carbidopa,
dans lequel l'inhibiteur de l'AADC est appliqué à raison d'une dose quotidienne de 300 à 1200 mg,
dans lequel ledit inhibiteur de l'AADC est fourni sous la forme d'une formulation orale à libération modifiée,
dans lequel la formulation orale à libération modifiée de l'inhibiteur de l'AADC libère constamment l'inhibiteur de l'AADC dans le tractus gastro-intestinal à raison de 12,5 à 50 mg/h pendant au moins 6 heures, et
dans lequel la formulation orale à libération modifiée de l'inhibiteur de l'AADC est appliquée 2 à 4 fois par jour,
b) une formulation liquide de lévodopa ou d'ester de lévodopa,
dans lequel la formulation liquide contient de la lévodopa ou de l'ester de lévodopa dans une molarité de 100 à 500 mmol/l,
dans lequel la formulation liquide est administrée par perfusion sous-cutanée,
dans lequel la dose quotidienne de lévodopa ou d'équivalent de lévodopa est comprise entre 100 et 900 mg,
c) éventuellement, une préparation pharmaceutique d'un inhibiteur de la COMT dans laquelle l'inhibiteur de la COMT est 25-75 mg d'opicapone.

2. Kit d'utilisation selon la revendication 1, dans lequel la formulation orale à libération modifiée de l'inhibiteur de l'AADC est fournie sous la forme d'un comprimé ou d'une capsule contenant chacun de 200 à 400 mg de bensérazide ou de carbidopa.

3. Kit d'utilisation selon la revendication 1, dans lequel la formulation orale à libération modifiée d'un inhibiteur de l'AADC est fournie sous la forme d'un comprimé ou d'une capsule contenant chacun de 200 à 400 mg de bensérazide.

4. Kit à utiliser selon l'une au moins des revendications 1 à 3, dans lequel l'ester de lévodopa est l'ester de pentaérythritol de la lévodopa.
